# EUROPÄISCHE PATENTSCHRIFT

(11) **EP 2 259 725 B1**
(45) Veröffentlichungstag und Bekanntmachung des Hinweises auf die Patenterteilung: **08.08.2012**
(21) Anmeldenummer: 09727011.0
(22) Anmeldetag: 30.03.2009
(51) Int. Cl.: A61B 6/00

(54) **ROENTGENVORRICHTUNG UND MEDIZINISCHER ARBEITSPLATZ**
X-RAY APPARATUS AND MEDICAL WORKSTATION
DISPOSITIF DE RADIOGRAPHIE ET POSTE DE TRAVAIL MEDICAL

(30) Priorität: 31.03.2008 DE 102008016414
(43) Veröffentlichungstag der Anmeldung: 15.12.2010
(73) Patentinhaber: KUKA Laboratories GmbH, 86165 Augsburg (DE)
(72) Erfinder: MUELLER, Michael, 47807 Krefeld (DE)
(74) Vertreter: Patentanwälte Funk & Böss GbR
(86) Internationale Anmeldenummer: PCT/EP2009/053704
(87) Internationale Veröffentlichungsnummer: WO 2009/121822

(56) Entgegenhaltungen:
- EP-A- 0 699 794
- EP-A- 1 004 271
- US-A1- 2003 091 156
- US-B1- 6 435 715

## Beschreibung

Die Erfindung betrifft eine Röntgenvorrichtung und einen medizinischen Arbeitsplatz.

Roboter im Allgemeinen sind Arbeitsmaschinen, die zur automatischen Handhabung und/ oder Bearbeiturig von Objekten mit Werkzeugen ausgerüstet werden können und in mehreren Beweguhgsachsen beispielsweise hinsichtlich Orientierung, Position und Arbeitsablauf programmierbar sind. Roboter weisen üblicherweise programmierbare Steuerungen (Steuerungsvorrichtungen) auf, die während des Betriebs die Bewegungsabläufe des Roboters steuern.

Die EP 1 106 141 A2 offenbart ein C-Bogen Röntgengerät, aufweisend einen C-Bogen, eine Röntgenquelle und einen Röntgendetektor, die an jeweils einem Ende des C-Bogens angeordnet sind. Der C-Bogen ist an einer als Roboterarm ausgebildeten Tragvorrichtung angeordnet.

Die EP 0 220 501 B1 offenbart eine Röntgendiagnostikanlage mit einer verstellbaren Röntgenröhre, einem verstellbaren Bildaufnahmesystem und einer Patientenliege als Anlagenkomponenten, die jeweils durch Tragarme gehalten sind. Die Tragarme sind nach Art von Roboterarmen aufgebaut und die Motoren der Tragarme sind mit einem zentrale Rechner verbunden, der Speichermittel für verschiedene feste Positionen und unterschiedliche Programmabläufe für kontinuierlich Bewegungen der Anlagenkomponenten aufweist.

Die EP 1 004 271 A1 offenbart eine Röntgenvorrichtung, die zwei Roboterarme und einen einzigen Steuerrechner aufweist, welcher die Bewegung beider Roboterarme steuert, indem er Signale erzeugt, die Reglern übergeben werden, welche wiederum die entsprechenden Roboterarme direkt bewegen.

Die US 6, 435,715 B1 und die US 2003/091156 A1 offenbaren weitere Röntgenvorrichtungen mit zwei Roboterarmen und mit einem gemeinsamen Steuerrechner.

Aufgabe der Erfindung ist es, eine flexibler aufgebaute Röntgenvorrichtung anzugeben, die eine an einem ersten Roboter angeordnete Röntgenstrahlenquelle und einen an einem zweiten Roboter angeordneten Röntgenstrahlenempfänger aufweist.

Die Aufgabe der Erfindung wird gelöst durch eine Röntgenvorrichtung, aufweisend
einen ersten Roboter, der mehrere erste Achsen und eine erste Befestigungsvorrichtung aufweist,
einen zweiten Roboter, der mehrere zweite Achsen und eine zweite Befestigungsvorrichtung aufweist,
eine an einer der beiden Befestigungsvorrichtungen angeordnete Röntgenstrahlenquelle und einen an der anderen Befestigungsvorrichtungen angeordneten Röntgenstrahlenempfänger,
wobei der erste Roboter einen ersten Steuerrechner aufweist, der eingerichtet ist, elektrische Antriebe des ersten Roboters anzusteuern, um die ersten Achsen für eine Bewegung der ersten Befestigungsvorrichtung anzusteuern, der zweiten Roboter einen zweiten Steuerrechner aufweist, der eingerichtet ist, elektrische Antriebe des zweiten Roboters anzusteuern, um die zweiten Achsen für eine Bewegung der zweiten Befestigungsvorrichtung anzusteuern, und die beiden Steuerrechner miteinander gekoppelt und als ein Master-Slave-System augebildet sind, bei dem der erste Steuerrechner als der Master und der zweite Steuerrechner als der Slave ausgebildet ist,
der erste Steuerrechner den zweiten Steuerrechner derart ansteuert, dass bei einer ersten Bewegung der ersten Befestigungsvorrichtung der zweite Steuerrechner die zweiten Achsen des zweiten Roboters derart bewegt, so dass die zweite Befestigungsvorrichtung eine zweite Bewegung ausführt, aufgrund derer die Röntgenstrahlenquelle und der Röntgenstrahlenempfänger stets zueinander in einem vorab festgelegten Abstand ausgerichtet sind.

Die erfindungsgemäße Röntgenvorrichtung weist demnach zwei Roboter auf, die jeweils mehrere Achsen und jeweils eine Befestigungsvorrichtung, insbesondere einen Flansch, umfassen. Einer der Roboter ist mit der Röntgenstrahlenquelle und der andere Roboter ist mit dem Röntgenstrahlenempfänger versehen. Im Vergleich zu einer Röntgenvorrichtung mit einer z.B. als C-Bogen ausgeführten Tragevorrichtung, an der die Röntgenstrahlenquelle und der Röntgenstrahlenempfänger befestigt sind und die von einem einzigen Roboter bewegt wird, ist die individuelle Traglast der beiden Roboter der erfindungsgemäßen Röntgenvorrichtung geringer.

Jeder der beiden Roboter umfasst seine eigene Steuerungsvorrichtung. Die jeweiligen Steuerungsvorrichtungen steuern im Betrieb der erfindungsgemäßen Röntgenvorrichtung die Achsen ihrer jeweiligen Roboter an. Dazu können, wie dies dem Fachmann allgemein bekannt ist, die Roboter mit elektrischen Antrieben versehen sein, die wiederum von den relevanten Steuerungsvorrichtungen angesteuert werden. Somit umfasst die erfindungsgemäße Röntgenvorrichtung keine zentrale Steuerungsvorrichtung, die alle Achsen beider Roboter zusammen ansteuert. Dadurch ist es möglich, zwei Standardroboter mit jeweils einer für sie bestimmten Steuerungsvorrichtung zu verwenden. Dies kann eine flexiblere Ausführung der erfindungsgemäßen Röntgenvorrichtung zur Folge haben.

Damit mittels der erfindungsgemäßen Röntgenvorrichtung Röntgenbilder von einem Objekt erstellt werden können, müssen die Röntgenstrahlenquelle und der Röntgenstrahlenempfänger im Betrieb zueinander in dem vorab festgelegten Abstand ausgerichtet sein. Wird die erfindungsgemäße Röntgenvorrichtung zudem im Betrieb bewegt, um z.B. eine Serie von zweidimensionalen Projektionen vom Objekt anzufertigen, dann ist es außerdem nötig, dass die Röntgenstrahlenquelle und der Röntgenstrahlenempfänger während dieser Bewegung möglichst stets zueinander ausgerichtet sind und den vorab festgelegten Abstand aufweisen. Um dies zu erreichen, sind die beiden Steuerungsvorrichtungen miteinander gekoppelt und als Master-Slave-System ausgebildet. Die erste Steuerungsvorrichtung ist dabei als Master und die zweite Steuerungsvorrichtung als Slave ausgebildet.

Im Betrieb der erfindungsgemäßen Röntgenvorrichtung ist es demnach möglich, dass die als Master ausgebildete Steuerungsvorrichtung (erste Steuerungsvorrichtung) während der ersten Bewegung der ersten Befestigungsvorrichtung des ersten Roboters die als Slave ausgebildete Steuerungsvorrichtung (zweite Steuerungsvorrichtung) ansteuert, so dass diese wiederum die zweiten Achsen des zweiten Roboters derart ansteuert, dass die zweite Befestigungsvorrichtung des zweiten Roboters der ersten Bewegung der ersten Befestigungsvorrichtung derart folgt, dass die Röntgenstrahlenquelle und der Röntgenstrahlenempfänger stets zueinander in dem vorab festgelegten Abstand ausgerichtet sind.

Gemäß einer Ausführungsform der erfindungsgemäßen Röntgenvorrichtung steuert die erste Steuerungsvorrichtung die ersten Achsen des ersten Roboters derart automatisch an, dass die erste Befestigungsvorrichtung die erste Bewegung durchführt. Um dies zu realisieren, läuft z.B. auf der ersten Steuerungsvorrichtung ein Rechenprogramm, aufgrund dessen die erste Steuerungsvorrichtung beispielsweise die oben erwähnten elektrischen Antriebe des ersten Roboters ansteuert, damit die erste Befestigungsvorrichtung und somit auch die Röntgenstrahlenquelle bzw. der Röntgenstrahlenempfänger, je nach dem welcher der beiden Komponenten an der ersten Befestigungsvorrichtung befestigt ist, die erste Bewegung automatisch durchführt.

Die erfindungsgemäße Röntgenvorrichtung kann auch mit der ersten Steuerungsvorrichtung gekoppelte Eingabemittel aufweisen, mittels deren die erste Steuerungsvorrichtung die ersten Achsen des ersten Roboters derart ansteuert, dass die erste Befestigungsvorrichtung die erste Bewegung aufgrund einer manuellen Eingabe in die Eingabemittel durchführt. Eingabemittel sind z.B. ein mit der ersten Steuerungsvorrichtung gekoppeltes Bediengerät, mittels dessen z.B. ein Arzt die Röntgenstrahlenquelle in eine gewünschte Position und Orientierung für eine Röntgenaufnahme bringen kann. Der Röntgenstrahlenempfänger wird dann mittels des zweiten Roboters automatisch derart ausgerichtet, dass er zur Röntgenstrahlenquelle im vorab festgelegten Abstand ausgerichtet ist.

Die erfindungsgemäße Röntgenvorrichtung kann auch derart ausgeführt sein, dass die erste Befestigungsvorrichtung die erste Bewegung aufgrund eines manuellen Bewegens der an der ersten Befestigungsvorrichtung angeordneten Röntgenstrahlenquelle bzw. des an der ersten Befestigungsvorrichtung angeordneten Röntgenstrahlenempfängers durchführt. Somit kann die erfindungsgemäße Röntgenvorrichtung durch manuelles Führen verstellt werden. Aufgrund der Maste-Slave Ausführung der beiden Steuerungsvorrichtungen steuert die zweite Steuerungsvorrichtung die zweite Befestigungsvorrichtung derart an, dass diese der manuellen Bewegung (erste Bewegung) der ersten Befestigungsvorrichtung automatisch folgt.

Die erste Bewegung kann einen ersten linearen Bewegungsanteil und/oder einen ersten Zirkularbewegungsanteil aufweisen und die zweite Steuerungsvorrichtung kann die zweiten Achsen derart bewegen, so dass die zweite Bewegung einen dem ersten linearen Bewegungsanteil entsprechenden zweiten linearen Bewegungsanteil und/oder einen dem ersten Zirkularbewegungsanteil entsprechenden zweiten Zirkularbewegungsanteil aufweist.

Gemäß einer Variante der erfindungsgemäßen Röntgenvorrichtung übermittelt die erste Steuerungsvorrichtung der zweiten Steuerungsvorrichtung während der ersten Bewegung eine Information über die aktuelle Position und Orientierung der ersten Befestigungsvorrichtung und die zweite Steuerungsvorrichtung bewegt aufgrund der relativen Lage und Orientierung des zweiten Roboters relativ zum ersten Roboter die zweiten Achsen des zweiten Roboters derart, so dass die zweite Befestigungsvorrichtung eine Position und Orientierung aufweist, in der die Röntgenstrahlenquelle und der Röntgenstrahlenempfänger während der ersten Bewegung stets zueinander in dem vorab festgelegten Abstand ausgerichtet sind. Die zweite Steuerungsvorrichtung umfasst nach dieser Variante eine Information über die relative Lage (Position und Orientierung) der beiden Roboter zueinander. Aufgrund der Information über die Lage (Position und Orientierung) der ersten Befestigungsvorrichtung ist es der zweiten Steuerungsvorrichtung möglich, die zweiten Achsen des zweiten Roboters derart zu verstellen, dass die zweite Befestigungsvorrichtung relativ zur ersten Befestigungsvorrichtung ausgerichtet ist, so dass die Röntgenstrahlenquelle und der Röntgenstrahlenempfänger während der ersten Bewegung stets zueinander in dem vorab festgelegten Abstand ausgerichtet sind. Die relative Lage der beiden Roboter zueinander kann beispielsweise durch Vermessen der beiden Roboter ermittelt werden.

Die erste Steuerungsvorrichtung kann der zweiten Steuerungsvorrichtung während der ersten Bewegung eine Information über die aktuelle Position und Orientierung der an der ersten Befestigungsvorrichtung angeordneten Röntgenstrahlenquelle bzw. des an der ersten Befestigungsvorrichtung Röntgenstrahlenempfängers übermitteln. Die zweite Steuerungsvorrichtung kann dann aufgrund der relativen Lage und Orientierung des zweiten Roboters relativ zum ersten Roboter und der Information über die aktuelle Position und Orientierung der an der ersten Befestigungsvorrichtung angeordneten Röntgenstrahlenquelle bzw. des an der ersten Befestigungsvorrichtung angeordneten Röntgenstrahlenempfängers die zweiten Achsen des zweiten Roboters derart bewegen, so dass die zweite Befestigungsvorrichtung eine Position und Orientierung aufweist, in der die Röntgenstrahlenquelle und der Röntgenstrahlenempfänger während der ersten Bewegung stets zueinander in dem vorab festgelegten Abstand ausgerichtet sind.

Der erste Roboter kann ein erstes Roboter-Basiskoordinatensystem und ein der ersten Befestigungsvorrichtung zugeordnetes Befestigungsvorrichtungskoordinatensystem aufweisen. Der zweite Roboter kann ein zweites Roboter-Basiskoordinatensystem aufweisen.

Nach einer Variante der erfindungsgemäßen Röntgenvorrichtung übermittelt die erste Steuerungsvorrichtung der zweiten Steuerungsvorrichtung während der ersten Bewegung eine Information über die aktuelle Position und Orientierung des Befestigungsvorrichtungskoordinatensystems und die zweite Steuerungsvorrichtung bewegt aufgrund der Information über die Position und Orientierung des Befestigungsvorrichtungskoordinatensystem und aufgrund einer Information über die relative Position und Orientierung der beiden Roboter-Basiskoordinatensysteme zueinander die zweiten Achsen des zweiten Roboters derart, so dass die zweite Befestigungsvorrichtung eine Position und Orientierung aufweist, in der die Röntgenstrahlenquelle und der Röntgenstrahlenempfänger während der ersten Bewegung stets zueinander in dem vorab festgelegten Abstand ausgerichtet sind.

Nach einer Variante der erfindungsgemäßen Röntgenvorrichtung ist die Röntgenstrahlenquelle an der ersten Befestigungsvorrichtung des ersten Roboters angeordnet ist und weist ein Röntgenstrahlenquellekoordinatensystem auf, ist der Röntgenstrahlenempfänger an der zweiten Befestigungsvorrichtung des zweiten Roboters angeordnet ist und weist einen Tool Center Point auf, steht der zweiten Steuerungsvorrichtung eine Information über die Beziehung zwischen dem ersten Befestigungsvorrichtungskoordinatensystem und dem Röntgenstrahlenquellekoordinatensystem zur Verfügung und ist die zweite Steuerungsvorrichtung eingerichtet, aufgrund der Information über die Position und Orientierung des Befestigungsvorrichtungskoordinatensystems, die Information über die Beziehung zwischen dem Befestigungsvorrichtungskoordinatensystem und dem Röntgenstrahlenquellekoordinatensystem und aufgrund der Information über die relative Position und Orientierung der beiden Roboter-Basiskoordinatensysteme zueinander die zweiten Achsen des zweiten Roboters derart bewegt, so dass der Tool Center Point derart ausgerichtet ist, dass die Röntgenstrahlenquelle und der Röntgenstrahlenempfänger während der ersten Bewegung stets zueinander im vorab festgelegten Abstand ausgerichtet sind.

Die Röntgenstrahlenquelle kann auch an der zweiten Befestigungsvorrichtung des zweiten Roboters und der Röntgenstrahlenempfänger kann an der ersten Befestigungsvorrichtung des ersten Roboters angeordnet sein, wobei dem Röntgenstrahlenempfänger ein Röntgenstrahlenempfängerkoordinatensystem zugeordnet ist und die Röntgenstrahlenquelle den Tool Center Point aufweist. Steht der zweiten Steuerungsvorrichtung eine Information über die Beziehung zwischen dem ersten Befestigungsvorrichtungskoordinatensystem und dem Röntgenstrahlenempfängerkoordinatensystem zur Verfügung, dann kann die zweite Steuerungsvorrichtung eingerichtet sein, aufgrund der Information über die Position und Orientierung des Befestigungsvorrichtungskoordinatensystems, die Information über die Beziehung zwischen dem Befestigungsvorrichtungskoordinatensystem und dem Röntgenstrahlenquellekoordinatensystem und aufgrund der Information über die relative Position und Orientierung der beiden Roboter-Basiskoordinatensysteme zueinander die zweiten Achsen des zweiten Roboters derart zu bewegen, so dass der Tool Center Point derart ausgerichtet ist, dass die Röntgenstrahlenquelle und der Röntgenstrahlenempfänger während der ersten Bewegung stets zueinander im vorab festgelegten Abstand ausgerichtet sind.

Es ist aber auch möglich, dass die erste Steuerungsvorrichtung der zweiten Steuerungsvorrichtung direkt aufgrund der Information über die Position und Orientierung des Röntgenstrahlenquellekoordinatensystems bzw. des Röntgenstrahlenempfängerkoordinatensystems die zweiten Achsen des Roboters derart bewegt, dass die Röntgenstrahlenquelle und der Röntgenstrahlenempfänger während der ersten Bewegung stets zueinander im vorab festgelegten Abstand ausgerichtet sind.

Die beiden Roboter können frei im Raum aufgestellt sein. Einer der Roboter oder auch beide können aber auch an einer Decke oder an einer Wand aufgehängt sein. Einer der Roboter oder beide der Roboter können auch mittels einer insbesondere Schienen gebundnen Lineareinheit bewegbar sein. Dadurch kann der Arbeitsbereich des relevanten Roboters vergrößert werden, wodurch sich die Flexibilität der erfindungsgemäßen Röntgenvorrichtung erhöhen kann.

Die erfindungsgemäße Röntgenvorrichtung ist insbesondere für einen medizinischen Arbeitsplatz vorgesehen, der neben der erfindungsgemäßen Röntgenvorrichtung eine Patientenliege aufweist.

Ausführungsbeispiele der Erfindung sind exemplarisch in den beigefügten schematischen Zeichnungen dargestellt. Es zeigen:
- Fig. 1: einen medizinischen Arbeitsplatz,
- Fig. 2: einen ersten Roboter, an dessen Flansch eine Röntgenstrahlenquelle befestigt ist,
- Fig. 3: einen zweiten Roboter, an dessen Flansch ein Röntgenstrahlenempfänger befestigt ist,
- Fig. 4: die am Flansch des ersten Roboters befestigte Röntgenstrahlenquelle und
- Fig. 5: der am Flansch des zweiten Roboters befestigte Röntgenstrahlenempfänger.

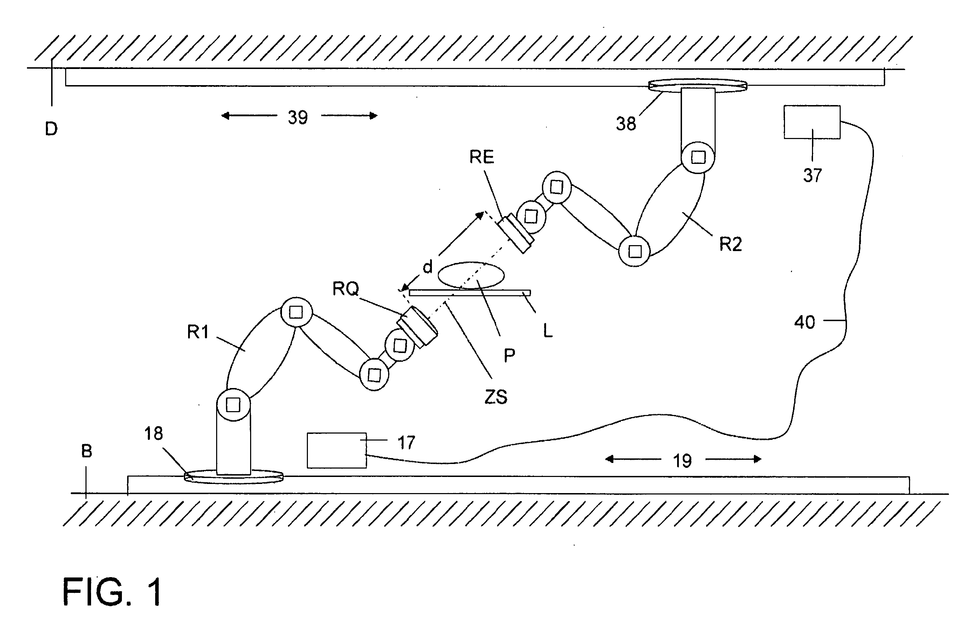

Die Fig. 1 zeigt einen medizinischen Arbeitsplatz mit einer Röntgenvorrichtung und einer nur schematisch angedeuteten Patientenliege L, auf der ein Lebewesen P für eine Untersuchung mit der Röntgenvorrichtung liegt.

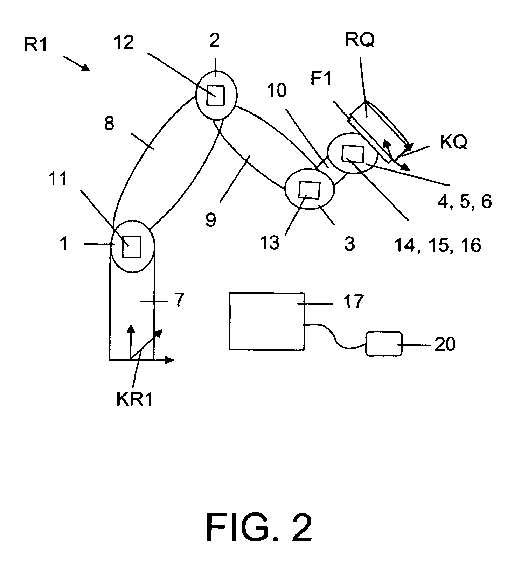

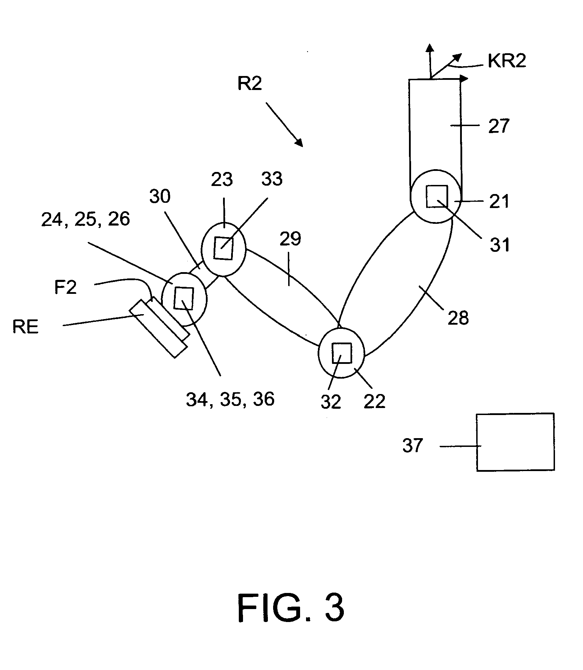

Die in der Fig. 1 gezeigte Röntgenvorrichtung weist einen in de Fig. 2 näher dargestellten ersten Roboter R1 und einen in der Fig. 3 näher dargestellten zweiten Roboter R2 auf.

Der erste Roboter R1 umfasst mehrere Achsen 1 - 6, mehrere Hebel 7 - 10 und einen Flansch F1, an dem eine Röntgenstrahlenquelle RQ befestigt ist. Jede der Achsen 1 - 6 wird im Falle des vorliegenden Ausführungsbeispieles mit einem elektrischen Antrieb bewegt, die in nicht dargestellter Weise mit einem Steuerrechner 17 des ersten Roboters R1 elektrisch verbunden sind, so dass der Steuerrechner 17 bzw. ein auf dem Steuerrechner 17 laufendes Rechnerprogramm die elektrischen Antriebe des ersten Roboters R1 derart ansteuern kann, dass die Position und Orientierung des Flansches F1 des ersten Roboters R1 im Wesentlichen frei im Raum ausgerichtet werden kann. Die elektrischen Antriebe des ersten Roboters R1 umfassen jeweils einen elektrischen Motor 11 - 16 und gegebenenfalls eine die Motoren 11 - 16 ansteuernde Leistungselektronik.

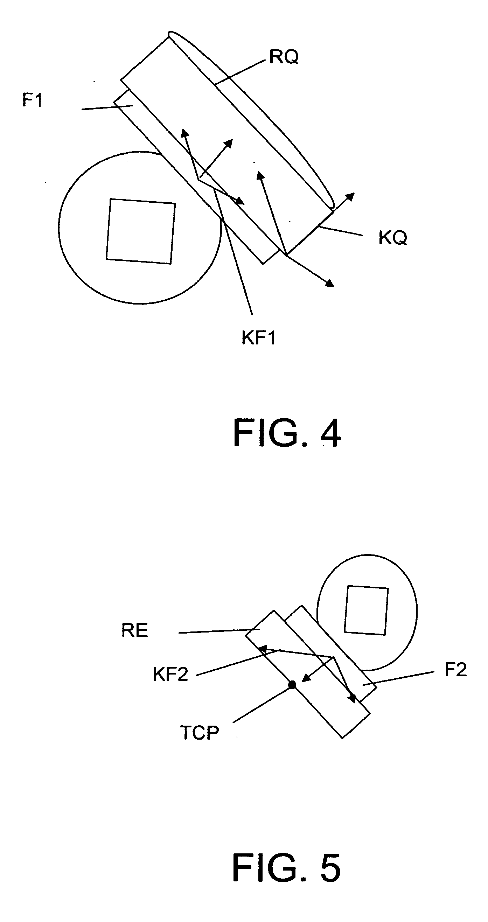

Der erste Roboter R1 weist ferner ein Roboterkoordinatensystem KR1 und der Flansch F1 des ersten Roboters R1 weist ein Flanschkoordinatensystem KF1 auf, das in der Fig. 4 dargestellt ist. Ferner weist die Röntgenstrahlenquelle RQ ein Röntgenstrahlenquellekoordinatensystem KQ auf.

Der zweite Roboter R2 umfasst mehrere Achsen 21 - 26, mehrere Hebel 27 - 30 und einen Flansch F2, an dem ein Röntgenstrahlenempfänger RE befestigt ist. Jede der Achsen 21 - 26 wird im Falle des vorliegenden Ausführungsbeispieles mit einem elektrischen Antrieb bewegt, die in nicht dargestellter Weise mit einem Steuerrechner 37 des zweiten Roboters R2 elektrisch verbunden sind, so dass der Steuerrechner 37 bzw. ein auf dem Steuerrechner 37 laufendes Rechnerprogramm die elektrischen Antriebe des zweiten Roboters R2 derart ansteuern kann, dass die Position und Orientierung des Flansches F2 des zweiten Roboters R2 im Wesentlichen frei im Raum ausgerichtet werden kann. Die elektrischen Antriebe des zweiten Roboters R2 umfassen jeweils einen elektrischen Motor 31 - 36 und gegebenenfalls eine die Motoren 31 - 36 ansteuernde Leistungselektronik.

Der zweite Roboter R2 weist ferner ein Roboterkoordinatensystem KR2 und der Flansch F2 des zweiten Roboters R2 weist ein Flanschkoordinatensystem KF2 auf, das in der Fig. 5 dargestellt ist. Ferner weist der Röntgenstrahlenempfänger RE einen Tool Center Point TCP auf und die beiden Steuerrechner 17, 37 sind mittels einer Datenleitung 40 verbunden.

Im Falle des vorliegenden Ausführungsbeispiels ist der erste Roboter R1 an einer am Boden B des medizinischen Arbeitsplatzes befestigten und insbesondere schienengebundenen Lineareinheit 18 befestigt, mittels derer der erste Roboter R1 entlang eines Doppelpfeils 19 bewegbar ist. Der zweite Roboter R2 ist an einer an der Decke D des medizinischen Arbeitsplatzes befestigten und insbesondere schienengebundenen Lineareinheit 38 befestigt, mittels derer der zweite Roboter R2 entlang eines Doppelpfeils 39 bewegbar ist. Die beiden Lineareinheiten 18, 38 umfassen jeweils in den Figuren nicht dargestellte Antriebe, wobei der Antrieb der ersten Lineareinheit 18 mit dem Steuerrechner 17 des ersten Roboters R1 und der Antrieb der zweiten Lineareinheit 38 mit dem Steuerrechner 37 des zweiten Roboters R2 verbunden ist. Im Betrieb der Roboter R1, R2 steuert der Steuerrechner 17 des ersten Roboters R1 die Lineareinheit 18, um den ersten Roboter R1 längs des Doppelpfeils 19 zu bewegen, und der Steuerrechner 37 des zweiten Roboters R2 steuert die Lineareinheit 38, um den zweiten Roboter R2 längs des Doppelpfeils 39 zu bewegen.

Im Falle des vorliegenden Ausführungsbeispiels kann in einem ersten Betriebsmodus z.B. ein in den Figuren nicht näher dargestellter Arzt den ersten Roboter R1 mittels eines mit dem ersten Steuerrechner 17 verbundenen Bediengeräts 20 derart bedienen, dass der Steuerrechner 17 die Antriebe des ersten Roboters R1 derart ansteuert, dass der Flansch F1 und somit die Röntgenstrahlenquelle RQ eine vom Arzt bestimmte Bewegung durchführt. Somit ist es dem Arzt möglich, die Röntgenstrahlenquelle RQ relativ zum Lebewesen P in gewünschter Weise auszurichten, um eine Röntgenaufnahme von einem Körperbereich des Lebewesens P herzustellen.

Für die Röntgenaufnahme, die der Arzt z.B. mittels eines nicht näher dargestellten Eingabemittels des Bediengerätes 20 auslösen kann, erzeugt die Röntgenstrahlenquelle RQ eine Röntgenstrahlung mit einem Zentralstrahl ZS.

Für die Aufnahme wird die Röntgenstrahlung beim Durchtritt durch das Lebewesen P teilweise geschwächt und trifft auf den Röntgenstrahlenempfänger RE auf. Dieser wandelt die auftreffende Röntgenstrahlung in ein der Röntgenstrahlung entsprechendes elektrisches Signal um, dem wiederum ein nicht näher dargestelltes Röntgenbild vom relevanten Körperbereich des Lebewesens P zugeordnet ist. Das Röntgenbild kann z.B. mittels eines der Übersicht halber nicht näher dargestellten Bildschirms betrachtet werden.

Damit das Röntgenbild eine zumindest zufriedenstellende Qualität aufweist, sollte während der Röntgenaufnahme der Röntgenstrahlenempfänger RE relativ zur Röntgenstrahlenquelle RQ in einem vorab festgelegten Abstand d ausgerichtet sein. Dies wird im Falle des vorliegenden Ausführungsbeispiels folgendermaßen realisiert:

Die beiden Steuerrechner 17, 37 sind als ein Master-Slave System ausgebildet, wobei im Falle des vorliegenden Ausführungsbeispiels der Steuerrechner 17 des ersten Roboters R1 als der Master und der Steuerrechner 37 des zweiten Roboters R2 als der Slave ausgebildet sind. Aufgrund der Eingabe in das Bediengerät 20 steuert der Steuerrechner 20 die elektrischen Antriebe des ersten Roboters R1 und gegebenenfalls der Lineareinheit 18 derart an, dass der Flansch F1 des ersten Roboters R1 und somit die Röntgenstrahlenquelle RQ die Bewegung ausführen. Gleichzeitig übermittelt der Steuerrechner 17 des ersten Roboters R1 dem Steuerrechner 37 des zweiten Roboters R2 eine Information über die aktuelle Position und Orientierung seines Flansches F1, die im Falle des vorliegenden Ausführungsbeispiels eine Information über die Position und Orientierung des Flanschkoordinatensystems KF1 darstellt. Dem Steuerrechner 37 des zweiten Roboters R2 steht außerdem eine Information über die relative Lage (Orientierung und Position) zwischen dem Flanschkoordinatensystem KF1 und dem Röntgenstrahlenquellekoordinatensystem KQ zur Verfügung. Ferner wurden die beiden Roboter R1, R2 vorab vermessen, so dass dem zweiten Roboter R2 auch eine Information über die relative Lage der beiden Roboter R1, R2 zueinander, insbesondere die relative Lage der beiden Roboterkoordinatensysteme KR1, KR2 bekannt ist. Eine Information über die aktuelle Position des mittels der Lineareinheit 18 verfahrbaren ersten Roboters R1 übermittelt der Steuerrechner 17 des ersten Roboters R1 ebenfalls dem Steuerrechner 37 des zweiten Roboters R2 während der Bewegung des Flansches F1 des ersten Roboters R1.

Somit ist es dem Steuerrechner 37 des zweiten Roboters R2 möglich, die aktuelle Position und Orientierung des Flansches F1 des ersten Roboters R1 bzw. der Röntgenstrahlenquelle RQ zu errechnen, um wiederum mittels eines auf dem Steuerrechner R2 des zweiten Roboters R2 laufenden Rechnerprogramms die Antriebe des zweiten Roboters R2 derart anzusteuern, dass der Flansch F2 des zweiten Roboters R2 und insbesondere der Tool Center Point TCP des Röntgenstrahlenempfängers RQ derart ausgerichtet wird, dass dieser den vorab festgelegten Abstand d zur Röntgenstrahlenquelle RQ aufweist und ebenfalls auf diese ausgerichtet ist.

Die oben beschriebenen Informationen übermittelt der Steuerrechner 17 des ersten Roboters R1 kontinuierlich dem Steuerrechner 37 des zweiten Roboters R2 während der Bewegung, so dass der Steuerrechner 37 des zweiten Roboters R2 stets die Antrieb des zweiten Roboters R2 derart ansteuern kann, damit der Röntgenstrahlenempfänger RE stets auf die Röntgenstrahlenquelle RQ im Abstand d ausgerichtet ist.

Im Falle des vorliegenden Ausführungsbeispiels kann die Röntgenvorrichtung in einem zweiten Betriebsmodus betrieben werden. Im zweiten Betriebsmodus kann der Arzt z.B. mittels des Bediengerätes 20 die Röntgenstrahlenquelle RQ wie gewünscht ausrichten. Alternativ ist es auch möglich, die Röntgenstrahlenquelle RQ beispielsweise durch manuelles Führen wie gewünscht auszurichten. Ist die Röntgenstrahlenquelle RQ wie gewünscht ausgerichtet, dann übermittelt der Steuerrechner R1 des ersten Roboters R1 dem Steuerrechner 37 des zweiten Roboters R2 die Information über die Lage des Flanschkoordinatensystems KF1 und die Position des ersten Roboters R1 bezüglich der Lineareinheit 18. Daraufhin steuert der Steuerrechner 37 des zweiten Roboters R2 die Antriebe des zweiten Roboters R2 derart an, dass der Röntgenstrahlenempfänger RE im Abstand d zur Röntgenstrahlenquelle RQ ausgerichtet wird.

Anschließend steuert der Steuerrechner R1 des ersten Roboters R1 die Antriebe des ersten Roboters R1 derart an, dass sich der Flansch F1 des ersten Roboters R1 und somit die Röntgenstrahlenquelle RQ auf einem Kreisbogen bewegen und im Falle des vorliegenden Ausführungsbeispiels in etwa einen Winkel von 190° überstreichen.

Während dieser Bewegung übermittelt der Steuerrechner 17 des ersten Roboters R1 kontinuierlich eine Information über die Position und Orientierung des Flanschkoordinatensystems KF1 sowie die Position des ersten Roboters R1 bezüglich der Lineareinheit 18.

Aufgrund dieser Information ist dem Steuerrechner 37 die Lage der Röntgenstrahlenquelle RQ während der Bewegung des Roboters R1 stets bekannt und kann demnach die Antriebe des zweiten Roboters R2 derart ansteuern, so dass der Tool Center Point TCP des Röntgenstrahlenempfängers RE im Abstand d derart zur Röntgenstrahlenquelle RQ stets ausgerichtet ist, dass die Röntgenstrahlenquelle RQ und der Röntgenstrahlenempfänger RE im Abstand d zueinender ausgerichtet sind.

Während der Bewegungen der Röntgenstrahlenquelle RQ und des Röntgenstrahlenempfängers RE um das Lebewesen P auf jeweils einem Kreisbogen mit einem Winkel von etwa 190° sendet die Röntgenstrahlenquelle RQ die Röntgenstrahlung mit dem Zentralstrahl ZS aus, damit der Röntgenstrahlenempfänger RE eine Serie von 2D-Bilddatensätzen erstellen kann. Aus den 2D-Bilddatensätzen kann dann in allgemein bekannter Weise ein Volumendatensatz vom Körperbereich des Lebewesens P berechnet werden.

Im Falle des vorliegenden Ausführungsbeispiels kann die Röntgenvorrichtung in einem dritten Betriebsmodus betrieben werden. Im dritten Betriebsmodus bewegt z.B. der Arzt die Röntgenstrahlenquelle RQ manuell z.B. durch Führen oder Ziehen am ersten Roboter R1 oder an der Röntgenstrahlenquelle RQ. Während des manuellen Bewegens übermittelt der Steuerrechner 17 des ersten Roboters R1 kontinuierlich eine Information über die Position und Orientierung des Flanschkoordinatensystems KF1 sowie die Position des ersten Roboters R1 bezüglich der Lineareinheit 18.

Aufgrund dieser Information ist dem Steuerrechner 37 die Lage der Röntgenstrahlenquelle RQ während der manuellen Bewegung des Roboters R1 stets bekannt und kann demnach die Antriebe des zweiten Roboters R2 derart ansteuern, so dass der Tool Center Point TCP des Röntgenstrahlenempfängers RE im Abstand d derart zur Röntgenstrahlenquelle RQ stets ausgerichtet ist, dass die Röntgenstrahlenquelle RQ und der Röntgenstrahlenempfänger RE im Abstand d zueinender ausgerichtet sind.

In den beschriebenen Ausführungsbeispielen übermittelt der Steuerrechner 17 des ersten Roboters R1 eine Information über die Lage des Flanschkoordinatensystems KF1 des ersten Roboters R1 an den Steuerrechner 37 des zweiten Roboters R2, der daraufhin die Lage der Röntgenstrahlenquelle RQ bzw. deren Röntgenstrahlenquellekoordinatensystem KQ berechnet. Es ist aber auch möglich, dass der Steuerrechner 17 des ersten Roboters R1 eine Information über die Lage der Röntgenstrahlenquelle RQ, insbesondere die Lage des Röntgenstrahlenquellekoordinatensystems KQ dem Steuerrechner 37 des zweiten Roboters R2 direkt übermittelt.

In den beschrieben Ausführungsbeispielen ist die Röntgenstrahlenquelle RQ am ersten Roboter R1 und der Röntgenstrahlenempfänger RE am zweiten Roboter R2 befestigt. Es ist aber auch möglich, dass der Röntgenstrahlenempfänger RE am ersten Roboter R1 und die Röntgenstrahlenquelle RQ am zweiten Roboter R2 befestigt ist, d.h. dass derjenige Roboter, an dem der Röntgenstrahlenempfänger RE befestigt ist, der Master ist.

## Patentansprüche

1. Röntgenvorrichtung, aufweisend
- einen ersten Roboter (R1), aufweisend mehrere erste Achsen (1-6) und eine erste Befestigungsvorrichtung (F1),
- einen zweiten Roboter (R2), aufweisend mehrere zweite Achsen (21-26) und eine zweite Befestigungsvorrichtung (F2),
- eine an einer der beiden Befestigungsvorrichtungen (F1) angeordnete Röntgenstrahlenquelle (RQ) und
- einen an der anderen Befestigungsvorrichtungen (F2) angeordneten Röntgenstrahlenempfänger (RE),
**dadurch gekennzeichnet, dass**
- der erste Roboter (R1) einen ersten Steuerrechner (17) aufweist, der eingerichtet ist, elektrische Antriebe des ersten Roboters (R1) anzusteuern, um die ersten Achsen (1-6) für eine Bewegung der ersten Befestigungsvorrichtung (F1) anzusteuern,
- der zweite Roboter (R2) einen zweiten Steuerrechner (37) aufweist, der eingerichtet ist, elektrische Antriebe des zweiten Roboters (R2) anzusteuern, um die zweiten Achsen (21-26) für eine Bewegung der zweiten Befestigungsvorrichtung (F2) anzusteuern,
wobei die beiden Steuerrechner (17, 37) miteinander gekoppelt und als ein Master-Slave-System ausgebildet sind, bei dem der erste Steuerrechner (17) als der Master und der zweite Steuerrechner (37) als der Slave ausgebildet ist, der erste Steuerrechner (17) den zweiten Steuerrechner (37) derart ansteuert, dass bei einer ersten Bewegung der ersten Befestigungsvorrichtung (F1) der zweite Steuerrechner (37) die zweiten Achsen (21-26) des zweiten Roboters (R2) derart bewegt, so dass die zweite Befestigungsvorrichtung (F2) eine zweite Bewegung ausführt, aufgrund derer die Röntgenstrahlenquelle (RQ) und der Röntgenstrahlenempfänger (RE) stets zueinander in einem vorab festgelegten Abstand (d) ausgerichtet sind.

2. Röntgenvorrichtung nach Anspruch 1, bei der
- der erste Steuerrechner (17) die ersten Achsen (1-6) des ersten Roboters (R1) derart automatisch ansteuert, dass die erste Befestigungsvorrichtung (F1) die erste Bewegung durchführt, und/oder
- die erste Befestigungsvorrichtung (F1) die erste Bewegung aufgrund eines manuellen Bewegens der an der ersten Befestigungsvorrichtung (F1) angeordneten Röntgenstrahlenquelle (RQ) bzw. des an der ersten Befestigungsvorrichtung (F1) angeordneten Röntgenstrahlenempfängers (RE) durchführt.

3. Röntgenvorrichtung nach Anspruch 1 oder 2, aufweisend mit dem ersten Steuerrechner (17) gekoppelte Eingabemittel (20), mittels deren der erste Steuerrechner (17) die ersten Achsen (1-6) des ersten Roboters (R1) derart ansteuert, dass die erste Befestigungsvorrichtung (F1) die erste Bewegung aufgrund einer manuellen Eingabe in die Eingabemittel (20) durchführt.

4. Röntgenvorrichtung nach einem der Ansprüche 1 bis 3, bei der
- der erste Steuerrechner (17) den zweiten Steuerrechner (37) während der ersten Bewegung eine Information über die aktuelle Position und Orientierung der ersten Befestigungsvorrichtung (F1) und/oder eine Information über die aktuelle Position und Orientierung der an der ersten Befestigungsvorrichtung (F1) angeordneten Röntgenstrahlenquelle (RQ) bzw. des an der ersten Befestigungsvorrichtung (F1) angeordneten Röntgenstrahlenempfängers (RE) übermittelt, und
- der zweite Steuerrechner (37) aufgrund der relativen Lage und Orientierung des zweiten Roboters (R2) relativ zum ersten Roboter (R1) und der Information über die aktuelle Position und Orientierung der ersten Befestigungsvorrichtung (F1) und/oder der Information über die aktuelle Position und Orientierung der an der ersten Befestigungsvorrichtung (F1) angeordneten Röntgenstrahlenquelle (RQ) bzw. des an der ersten Befestigungsvorrichtung (F2) angeordneten Röntgenstrahlenempfängers (RE) die zweiten Achsen (21-26) des zweiten Roboters (R2) derart bewegt, so dass die zweite Befestigungsvorrichtung (F2) eine Position und Orientierung aufweist, in der die Röntgenstrahlenquelle (RQ) und der Röntgenstrahlenempfänger (RE) während der ersten Bewegung stets zueinander in dem vorab festgelegten Abstand (d) ausgerichtet sind.

5. Röntgenvorrichtung nach Anspruch 4, bei der
- der erste Roboter (R1) ein erstes Roboter-Basiskoordinatensystem (KR1) und der zweite Roboter (R2) ein zweites Roboter-Basiskoordinatensystem (KR2) aufweist,
- der erste Roboter (R1) ein der ersten Befestigungsvorrichtung (F1) zugeordnetes Befestigungsvorrichtungskoordinatensystem (KF1) aufweist,
- der erste Steuerrechner (17) den zweiten Steuerrechner (37) während der ersten Bewegung eine Information über die Position und Orientierung des Befestigungsvorrichtungskoordinatensystems (KF1) übermittelt und
- der zweite Steuerrechner (37) aufgrund der Information über die Position und Orientierung des Befestigungsvorrichtungskoordinatensystems (KF1) und aufgrund einer Information über die relative Position und Orientierung der beiden Roboter-Basiskoordinatensysteme zueinander (KR1, KR2) die zweiten Achsen (21-26) des zweiten Roboters (R2) derart bewegt, so dass die zweite Befestigungsvorrichtung (F2) eine Position und Orientierung aufweist, in der die Röntgenstrahlenquelle (RQ) und der Röntgenstrahlenempfänger (RE) während der ersten Bewegung stets zueinander in dem vorab festgelegten Abstand (d) ausgerichtet sind.

6. Röntgenvorrichtung nach Anspruch 5, bei der
- die Röntgenstrahlenquelle (RQ) an der ersten Befestigungsvorrichtung (F1) des ersten Roboters (R1) angeordnet ist und ein Röntgenstrahlenquellekoordinatensystem (KQ) aufweist,
- der Röntgenstrahlenempfänger (RE) an der zweiten Befestigungsvorrichtung (F2) des zweiten Roboters (R2) angeordnet ist und einen Tool Center Point (TCP) aufweist,
- dem zweiten Steuerrechner (37) eine Information über die Beziehung zwischen dem ersten Befestigungsvorrichtungskoordinatensystem (KF1) und dem Röntgenstrahlenquellekoordinatensystem (KQ) zur Verfügung steht und
- der zweite Steuerrechner (37) aufgrund der Information über die Position und Orientierung des Befestigungsvorrichtungskoordinatensystems (KF1), die Information über die Beziehung zwischen dem Befestigungsvorrichtungskoordinatensystem (KF1) und dem Röntgenstrahlenquellekoordinatensystem (KQ) und aufgrund der Information über die relative Position und Orientierung der beiden Roboter-Basiskoordinatensysteme (KR1, KR2) zueinander die zweiten Achsen (21-26) des zweiten Roboters (R2) derart bewegt, so dass der Tool Center Point (TCP) derart ausgerichtet ist, dass die Röntgenstrahlenquelle (RQ) und der Röntgenstrahlenempfänger (RE) während der ersten Bewegung stets zueinander im vorab festgelegten Abstand (d) ausgerichtet sind.

7. Röntgenvorrichtung nach Anspruch 5, bei der
- der Röntgenstrahlenempfänger (RE) an der ersten Befestigungsvorrichtung (F1) des ersten Roboters (R1) angeordnet ist und ein Röntgenstrahlenempfängerkoordinatensystem aufweist,
- die Röntgenstrahlenquelle (RQ) an der zweiten Befestigungsvorrichtung (F2) des zweiten Roboters (R2) angeordnet ist und einen Tool Center Point aufweist,
- dem zweiten Steuerrechner (37) eine Information über die Beziehung zwischen dem Befestigungsvorrichtungskoordinatensystem und dem Röntgenstrahlenempfängerkoordinatensystem zur Verfügung steht und
- der zweite Steuerrechner (37) aufgrund der Information über die Position und Orientierung des Befestigungsvorrichtungskoordinatensystems, die Information über die Beziehung zwischen dem Befestigungsvorrichtungskoordinatensystem und dem Röntgenstrahlenempfängerkoordinatensystem und aufgrund der Information über die relative Position und Orientierung der beiden Roboter-Basiskoordinatensysteme (KR1, KR2) zueinander die zweiten Achsen (21-26) des zweiten Roboters (R2) derart bewegt, so dass der Tool Center Point derart ausgerichtet ist, dass die Röntgenstrahlenquelle (RQ) und der Röntgenstrahlenempfänger (RE) während der ersten Bewegung stets zueinander im vorab festgelegten Abstand (d) ausgerichtet sind.

8. Röntgenvorrichtung nach Anspruch 4, bei der
- der erste Roboter (R1) ein erstes Roboter-Basiskoordinatensystem (KR1) und der zweite Roboter (R2) ein zweites Roboter-Basiskoordinatensystem (KR2) aufweist,
- die Röntgenstrahlenquelle (RQ) an der ersten Befestigungsvorrichtung (F1) des ersten Roboters (R1) angeordnet ist und ein Röntgenstrahlenquellekoordinatensystem (KQ) aufweist,
- der Röntgenstrahlenempfänger (RE) an der zweiten Befestigungsvorrichtung (F2) des zweiten Roboters (R2) angeordnet ist und einen Tool Center Point (TCP) aufweist,
- der erste Steuerrechner (17) den zweiten Steuerrechner (37) während der ersten Bewegung eine Information über die Position und Orientierung des Röntgenstrahlenquellekoordinatensystems (KQ) übermittelt und
- der zweite Steuerrechner (17) aufgrund der Information über die Position und Orientierung des Röntgenstrahlenquellekoordinatensystems (KQ) und aufgrund der Information über die relative Position und Orientierung der beiden Roboter-Basiskoordinatensysteme (KR1, KR2) zueinander die zweiten Achsen (21-26) des zweiten Roboters (R2) derart bewegt, so dass der Tool Center Point (TCP) derart ausgerichtet ist, dass die Röntgenstrahlenquelle (RQ) und der Röntgenstrahlenempfänger (RE) während der ersten Bewegung stets zueinander im vorab festgelegten Abstand (d) ausgerichtet sind.

9. Röntgenvorrichtung nach Anspruch 4, bei der
- der erste Roboter (R1) ein erstes Roboter-Basiskoordinatensystem (KR1) und der zweite Roboter (R2) ein zweites Roboter-Basiskoordinatensystem (KR2) aufweist,
- der Röntgenstrahlenempfänger (RE) an der ersten Befestigungsvorrichtung (F1) des ersten Roboters (R1) angeordnet ist und ein Röntgenstrahlenempfängerkoordinatensystem aufweist,
- die Röntgenstrahlenquelle (RQ) an der zweiten Befestigungsvorrichtung (F2) des zweiten Roboters angeordnet ist und einen Tool Center Point aufweist,
- der erste Steuerrechner (17) der zweiten Steuerrechner (37) während der ersten Bewegung eine Information über die Position und Orientierung des Röntgenstrahlenempfängerkoordinatensystem übermittelt und
- der zweite Steuerrechner (37) aufgrund der Information über die Position und Orientierung des Röntgenstrahlenempfängerkoordinatensystems und aufgrund der Information über die relative Position und Orientierung der beiden Roboter-Basiskoordinatensysteme (KR1, KR2) zueinander die zweiten Achsen (21-26) des zweiten Roboters (R2) derart bewegt, so dass der Tool Center Point derart ausgerichtet ist, dass die Röntgenstrahlenquelle (RQ) und der Röntgenstrahlenempfänger (RE) während der ersten Bewegung stets zueinander im vorab festgelegten Abstand (d) ausgerichtet sind.

10. Medizinischer Arbeitsplatz, aufweisend eine Röntgenvorrichtung nach einem der Ansprüche 1 bis 9 und eine Patientenliege (L).

## Claims

1. An X-ray device, comprising
- a first robot (R1) comprising a plurality of first axes (1-6) and a first attachment device (F1),
- a second robot (R2) comprising a plurality of second axes (21-26) and a second attachment device (F2),
- an X-ray source (RQ) arranged on one of the two attachment devices (F1) and
- an X-ray receiver (RE) arranged on the other attachment device (F2),
**characterised in that**
- the first robot (R1) comprises a first control computer (17) which is configured to control electrical drives of the first robot (R1), in order to control the first axes (1-6) for the movement of the first attachment device (F1),
- the second robot (R2) comprises a second control computer (37) which is configured to control electrical drives of the second robot (R2) in order to control the second axes (21-26) for the movement of the second attachment device (F2),
wherein the two control computers (17, 37) are coupled together and configured as a master-slave system, in which the first control computer (17) is configured as the master and the second control computer (37) is configured as the slave, the first control computer (17) controls the second control computer (37) such that with a first movement of the first attachment device (F1) the second control computer (37) moves the second axes (21-26) of the second robot (R2) such that the second attachment device (F2) executes a second movement, on the basis of which the X-ray source (RQ) and the X-ray receiver (RE) are continually aligned to one another at a previously defined spacing (d).

2. X-ray device according to claim 1, in which
- the first control computer (17) automatically controls the first axes (1-6) of the first robot (R1) such that the first attachment device (F1) performs the first movement and/or
- the first attachment device (F1) performs the first movement on the basis of a manual movement of the X-ray source (RQ) arranged on the first attachment device (F1) or of the X-ray receiver (RE) arranged on the first attachment device (F1).

3. X-ray device according to claim 1 or 2, comprising input means (20) coupled to the first control computer (17), by means of which the first control computer (17) controls the first axes (1-6) of the first robot (R1) such that the first attachment device (F1) performs the first movement on the basis of manual input into the input means (20).

4. X-ray device according to any one of claims 1 to 3 in which
- the first control computer (17) transmits to the second control computer (37) during the first movement information about the current position and orientation of the first attachment device (F1) and/or information about the current position and orientation of the X-ray source (RQ) arranged on the first attachment device (F1) or of the X-ray receiver (RE) arranged on the first attachment device (F1), and
- on the basis of the relative position and orientation of the second robot (R2) relative to the first robot (R1) and information about the current position and orientation of the first attachment device (F1) and/or information about the current position and orientation of the X-ray source (RQ) arranged on the first attachment device (F1) or the X-ray receiver (RE) arranged on the first attachment device (F2) the second control computer (37) moves the second axes (21-26) of the second robot (R2) such that the second attachment device (F2) has a position and orientation in which the X-ray source (RQ) and the X-ray receiver (RE) are continually aligned to one another at the previously defined spacing (d) during the first movement.

5. X-ray device according to claim 4, in which
- the first robot (R1) has a first robot basic coordinate system (KR1) and the second robot (R2) has a second robot basic coordinate system (KR2),
- the first robot (R1) has an attachment device coordinate system (KF1) assigned to the first attachment device (F1),
- the first control computer (17) transmits to the second control computer (37) during the first movement information about the position and orientation of the attachment device coordinate system (KF 1) and
- on the basis of information on the position and orientation of the attachment device coordinate system (KF 1) and on the basis of information on the relative position and orientation of the two robot basis coordinate systems to one another (KR1, KR2) the second control computer (37) moves the second axes (21-26) of the second robot (R2) such that the second attachment device (F2) has a position and orientation in which the X-ray source (RQ) and the X-ray receiver (RE) are continually aligned to one another at the previously determined spacing (d) during the first movement.

6. X-ray device according to claim 5, in which
- the X-ray source (RQ) is arranged on the first attachment device (F1) of the first robot (R1) and comprises an X-ray source coordinate system (KQ),
- the X-ray receiver (RE) is arranged on the second attachment device (F2) of the second robot (R2) and comprises a tool centre point (TCP),
- information on the relationship between the first attachment device coordinate system (KF1) and the X-ray source coordinate system (KQ) is available to the second control computer (37) and
- on the basis of information on the position and orientation of the attachment device coordinate system (KF1), information on the relationship between the attachment device coordinate system (KF1) and the X-ray source coordinate system (KQ) and on the basis of information on the relative position and orientation of the two robot basic coordinate systems (KR1, KR2) to one another the second control computer (37) moves the second axes (21-26) of the second robot (R2) such that the tool centre point (TCP) is aligned so that the X-ray source (RQ) and the X-ray receiver (RE) are continually aligned to one another at a previously defined spacing (d) during the first movement.

7. X-ray device according to claim 5, in which
- the X-ray receiver (RE) is arranged on the first attachment device (F1) of the first robot (R1) and comprises an X-ray receiver coordinate system,
- the X-ray source (RQ) is arranged on the second attachment device (F2) of the second robot (R2) and comprises a tool centre point,
- information on the relationship between the attachment device coordinate system and the X-ray receiver coordinate system is available to the second control computer (37) and
- on the basis of information on the position and orientation of the attachment device coordinate system, information on the relationship between the attachment device coordinate system and the X-ray receiver coordinate system and on the basis of information on the relative position and orientation of the two robot basic coordinate systems (KR1, KR2) to one another the second control computer (37) moves the second axes (21-26) of the second robot (R2) such that the tool centre point (TCP) is aligned so that the X-ray source (RQ) and the X-ray receiver (RE) are continually aligned to one another at a previously defined spacing (d) during the first movement.

8. X-ray device according to claim 4, in which
- the first robot (R1) has a first robot basic coordinate system (KR1) and the second robot (R2) has a second robot basic coordinate system (KR2),
- the X-ray source (RQ) is located on the first attachment device (F1) of the first robot (R1) and comprises an X-ray source coordinate system (KQ),
- the X-ray receiver (RE) is located on the second attachment device (F2) of the second robot (R2) and has a tool centre point (TCP),
- the first control computer (17) transmits to the second control computer (37) during the first movement information on the position and orientation of the X-ray source coordinate system (KQ) and
- the second control computer (17) moves the second axes (21-26) of the second robot (R2) on the basis of the information on the position and orientation of the X-ray source coordinate system (KQ) and on the basis of information on the relative position and orientation of the two robot basic coordinate systems (KR1, KR2) relative to each other, such that the tool centre point (TCP) is oriented so that the X-ray source (RQ) and the X-ray receiver (RE) are continually aligned relative to each other at a predefined spacing (d) during the first movement.

9. The X-ray device according to claim 4, wherein
- the first robot (R1) has a first robot basic coordinate system (KR1) and the second robot (R2) has a second robot basic coordinate system (KR2),
- the X-ray receiver (RE) is situated on the first attachment device (F1) of the first robot (R1) and has an X-ray receiver coordinate system,
- the X-ray source (RQ) is situated on the second attachment device (F2) of the second robot and has a tool centre point,
- the first control computer (17) transmits to the second control device (37) information about the position and orientation of the X-ray receiver coordinate system during the first movement and
- the second control computer (37) moves the second axes (21-26) of the second robot (R2) on the basis of information on the position and orientation of the X-ray receiver coordinate system and on the basis of information on the relative position and orientation of the two robot basic coordinate systems (KR1, KR2) relative to each other, in such a way that the tool centre point (TCP) is oriented so that the X-ray source (RQ) and the X-ray receiver (RE) are continually aligned relative to one other at a predefined distance (d) during the first movement.

10. Medical workstation comprising an X-ray device according to any one of claims 1 to 9 and a patient transport trolley (L).

## Revendications

1. Dispositif de radiographie présentant
- un premier robot (R1) qui présente plusieurs premiers axes (1-6) et un premier dispositif de fixation (F1),
- un deuxième robot (R2) qui présente plusieurs deuxièmes axes (21-26) et un deuxième dispositif de fixation (F2),
- une source de rayons X (RQ) agencée sur l'un des deux dispositifs de fixation (F1) et
- un récepteur de rayons X (RE) agencé sur l'autre dispositif de fixation (F2),
**caractérisé en ce que**
- le premier robot (R1) présente un premier ordinateur de commande (17) qui est conçu pour piloter des entraînements électriques du premier robot (R1) pour piloter les premiers axes (1-6) pour un déplacement du premier dispositif de fixation (F1),
- le deuxième robot (R1) présente un deuxième ordinateur de commande qui est conçu pour piloter des entraînements électriques du deuxième robot (R2) pour piloter les deuxièmes axes (21-26) pour un déplacement du deuxième dispositif de fixation (F2),
les deux ordinateurs de commande (17, 37) étant couplés l'un à l'autre et réalisés sous forme de système maître-esclave dans lequel le premier ordinateur de commande (17) est réalisé en tant que le maître et le deuxième ordinateur de commande (37) en tant que l'esclave, le premier ordinateur de commande (17) pilotant le deuxième ordinateur de commande (37) de telle sorte qu'en cas d'un premier déplacement du premier dispositif de fixation (F1), le deuxième ordinateur de commande (37) déplace les deuxièmes axes (21-26) du deuxième robot (R2) de telle sorte que le deuxième dispositif de fixation(F2) exécute un deuxième déplacement à la base duquel la source de rayons X (RQ) et le récepteur de rayons X (RE) sont toujours orientés l'une vers l'autre à une distance (d) prédéterminée.

2. Dispositif de radiographie selon la revendication 1, dans lequel
- le premier ordinateur de commande (17) pilote automatiquement les premiers axes (1-6) du premier robot (R1) de telle sorte que le premier dispositif de fixation (F1) exécute le premier déplacement, et/ou
- le premier dispositif de fixation (F1) exécute le premier déplacement sur la base d'un déplacement manuel de la source de rayons X (RQ) agencée sur le premier dispositif de fixation (F1) ou du récepteur de rayons X (RE) agencé sur le premier dispositif de fixation (F1).

3. Dispositif de radiographie selon la revendication 1 ou 2, présentant des moyens d'entrée (20) couplés au premier ordinateur de commande (17) au moyen desquels le premier ordinateur de commande (17) pilote les premiers axes (1-6) du premier robot (R1) de telle sorte que le premier dispositif de fixation (F1) exécute le premier déplacement sur la base d'une entrée manuelle dans les moyens d'entrée (20).

4. Dispositif de radiographie selon l'une des revendications 1 à 3, dans lequel
- le premier ordinateur de commande (17) transmet au deuxième ordinateur de commande (37) pendant le premier déplacement une information sur la position et l'orientation actuelles de la source de rayons X (RQ) agencée sur le premier dispositif de fixation (F1) ou du récepteur de rayons X (RE) agencé sur le premier dispositif de fixation (F1), respectivement, et
- le deuxième ordinateur de commande (37), sur la base de la position et de l'orientation relatives du deuxième robot (R2) par rapport au premier robot (R1) et de l'information sur la position et l'orientation actuelles de la source de rayons X (RQ) agencée sur le premier dispositif de fixation (F1) ou du récepteur de rayons X (RE) agencé sur le premier dispositif de fixation (F1), respectivement, déplace les deuxièmes axes (21-26) du deuxième robot (R2) de telle sorte que le deuxième dispositif de fixation (F2) présente une position et une orientation dans lesquelles, pendant le premier déplacement, la source de rayons X (RQ) et le récepteur de rayons X (RE) sont toujours orientés l'une vers l'autre à la distance (d) prédéterminée.

5. Dispositif de radiographie selon la revendication 4, dans lequel
- le premier robot (R1) présente un premier système de coordonnées de base de robot (KR1) et le deuxième robot (R2) présente un deuxième système de coordonnées de base de robot (KR2),
- le premier robot (R1) présente un système de coordonnées de dispositif de fixation (KF1) associé au premier dispositif de fixation (F1),
- le premier ordinateur de commande (17) transmet au deuxième ordinateur de commande (37) pendant le premier déplacement une information sur la position et l'orientation du système de coordonnées de dispositif de fixation (KF1) et
- le deuxième ordinateur de commande (37), sur la base de l'information sur la position et l'orientation du système de coordonnées du dispositif de fixation (KF1) et sur la base d'une information sur la position et l'orientation relatives des deux systèmes de coordonnées de base de robot (KR1, KR2) l'un par rapport à l'autre, déplace les deuxièmes axes (21-26) du deuxième robot (R2) de telle sorte que le dispositif de fixation (F2) présente une position et une orientation dans lesquelles la source de rayons X (RQ) et le récepteur de rayons X (RE) sont, pendant le premier déplacement, toujours orientés l'une vers l'autre à la distance (d) prédéterminée.

6. Dispositif de radiographie selon la revendication 5, dans lequel
- la source de rayons X (RQ) est agencée sur le premier dispositif de fixation (F1) du premier robot (R1) et présente un système de coordonnées de source de rayons X (KQ),
- le récepteur de rayons X (RE) est agencé sur le deuxième dispositif de fixation (F2) du deuxième robot (R2) et présente un point centre outil (TCP),
- le deuxième ordinateur de commande (37) dispose d'une information sur le rapport entre le premier système de coordonnées de source de rayons X (KQ) et le système de coordonnées de source de rayons X (KQ) et
- le deuxième ordinateur de commande (37), sur la base de l'information sur la position et l'orientation du système de coordonnées du dispositif de fixation (KF1) et sur la base de l'information sur le rapport entre le système de coordonnées du dispositif de fixation (KF1) et le système de coordonnées de source de rayons X (KQ) et sur la base de l'information sur la position et l'orientation relatives des deux systèmes de coordonnées de base de robot (KR1, KR2) l'un par rapport à l'autre, déplace les deuxièmes axes (21-26) du deuxième robot (R2) de telle sorte que le point centre outil (TCP) est orienté de telle manière que la source de rayons X (RQ) et le récepteur de rayons X (RE) sont, pendant le premier déplacement, toujours orientés l'une vers l'autre à une distance (d) prédéterminée.

7. Dispositif de radiographie selon la revendication 5, dans lequel
- le récepteur de rayons X (RE) est agencé sur le premier dispositif de fixation (F1) du premier robot (R1) et présente un système de coordonnées de récepteur de rayons X,
- la source de rayons X (RQ) est agencé sur le deuxième dispositif de fixation (F2) du deuxième robot (R2) et présente un point centre outil,
- le deuxième ordinateur de commande (37) dispose d'une information sur le rapport entre le système de coordonnées de dispositif de fixation et le système de coordonnées de récepteur de rayons X et
- le deuxième ordinateur de commande (37), sur la base de l'information sur la position et l'orientation du système de coordonnées du dispositif de fixation et sur la base de l'information sur le rapport entre le système de coordonnées du dispositif de fixation et le système de coordonnées de récepteur de rayons X (KQ) et sur la base de l'information sur la position et l'orientation relatives des deux systèmes de coordonnées de base de robot (KR1, KR2) l'un par rapport à l'autre, déplace les deuxièmes axes (21-26) du deuxième robot (R2) de telle sorte que le point centre outil est orienté de telle manière que la source de rayons X (RQ) et le récepteur de rayons X (RE) sont, pendant le premier déplacement, toujours orientés l'une vers l'autre à une distance (d) prédéterminée.

8. Dispositif de radiographie selon la revendication 4, dans lequel
- le premier robot (R1) présente un premier système de coordonnées de base de robot (KR1) et le deuxième robot (R2) présente un deuxième système de coordonnées de base de robot (KR2),
- la source de rayons X (RQ) est agencée sur le premier dispositif de fixation (F1) du premier robot (R1) et présente un système de coordonnées de récepteur de rayons X (KQ),
- le récepteur de rayons X (RE) est agencé sur le deuxième dispositif de fixation (F2) du deuxième robot (R2) et présente un point centre outil (TCP),
- le premier ordinateur de commande (17) transmet au deuxième ordinateur de commande (37) pendant le premier déplacement une information sur la position et l'orientation du système de coordonnées de récepteur de rayons X (KQ), et
- le deuxième ordinateur de commande (37), sur la base de l'information sur la position et l'orientation du système de coordonnées de la source de rayons X (KQ) et sur la base de l'information sur la position et l'orientation relatives des deux systèmes de coordonnées de base de robot (KR1, KR2) l'un par rapport à l'autre, déplace les deuxièmes axes (21-26) du deuxième robot (R2) de telle sorte que le point centre outil (TCP) est orienté de telle manière que la source de rayons X (RQ) et le récepteur de rayons X (RE) sont, pendant le premier déplacement, toujours orientés l'une vers l'autre à une distance (d) prédéterminée.

9. Dispositif de radiographie selon la revendication 4, dans lequel
- le premier robot (R1) présente un premier système de coordonnées de base de robot (KR1) et le deuxième robot (R2) présente un deuxième système de coordonnées de base de robot (KR2),
- le récepteur de rayons X (RE) est agencé sur le premier dispositif de fixation (F1) du premier robot (R1) et présente un système de coordonnées de récepteur de rayons X (KQ),
- la source de rayons X (RQ) est agencée sur le deuxième dispositif de fixation (F2) du deuxième robot (R2) et présente un point centre outil (TCP)
- le premier ordinateur de commande (17) transmet au deuxième ordinateur de commande (37) pendant le premier déplacement une information sur la position et l'orientation du système de coordonnées de récepteur de rayons X, et
- le deuxième ordinateur de commande (37), sur la base de l'information sur la position et l'orientation du système de coordonnées de la source de rayons X (KQ) et sur la base de l'information sur la position et l'orientation relatives des deux systèmes de coordonnées de base de robot (KR1, KR2) l'un par rapport à l'autre, déplace les deuxièmes axes (21-26) du deuxième robot (R2) de telle sorte que le point centre outil (TCP) est orienté de telle manière que la source de rayons X (RQ) et le récepteur de rayons X (RE) sont, pendant le premier déplacement, toujours orientés l'une vers l'autre à une distance (d) prédéterminée.

10. Poste de travail médical présentant un dispositif de radiographie selon l'une des revendications 1 à 9 et une table de support de patient (L).
